(19) Europäisches Patentamt
European Patent Office
Office européen des brevets

(11) Veröffentlichungsnummer: **0 216 028**
**A2**

(12) # EUROPÄISCHE PATENTANMELDUNG

(21) Anmeldenummer: 86108696.5

(22) Anmeldetag: 26.06.86

(51) Int.Cl.[4]: A 61 K 31/18
C 07 C 143/79, C 07 C 147/1-3
C 07 C 147/14, C 07 C 149/4-3

(30) Priorität: 03.07.85 DE 3523705

(43) Veröffentlichungstag der Anmeldung:
01.04.87 Patentblatt 87/14

(84) Benannte Vertragsstaaten:
AT BE CH DE FR GB IT LI LU NL SE

(71) Anmelder: HOECHST AKTIENGESELLSCHAFT
Postfach 80 03 20
D-6230 Frankfurt am Main 80(DE)

(72) Erfinder: Lohaus, Gerhard, Dr.
Uhlandstrasse 4
D-6233 Kelkheim (Taunus)(DE)

(72) Erfinder: Loewe, Heinz, Dr.
Berliner Ring 6
D-6233 Kelkheim (Taunus)(DE)

(72) Erfinder: Düwel, Dieter, Dr.
Frankfurter Strasse 39
D-6238 Hofheim am Taunus(DE)

(54) **Verwendung von Anthranilsäure-Derivaten als anthelmintische Arzneimittel.**

(57) Arzneimittel, gekennzeichnet durch einen Gehalt an Verbindungen der allgemeinen Struktur I

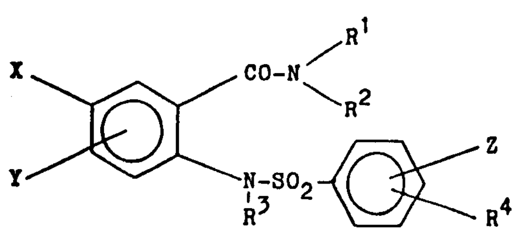

(I)

worin X Halogen, $CF_3$, $NO_2$, $SO_nR$ mit $R=CH_3$ oder $C_2H_5$, $n=0-2$ bedeutet, Y Wasserstoff oder Halogen, $R^1$ einen durch mindestens ein Halogenatom oder eine $CF_3$- oder $OCF_3$-Gruppe substituierten Phenylrest oder einen Alkylrest mit 6-18 Kohlenstoffatomen, $R^2$ Wasserstoff oder einen Alkylrest mit 1 bis 8 Kohlenstoffatomen bedeuten, oder $R^1$ und $R^2$ zusammen einen gesättigten oder äthylenisch ungesättigten Kohlenwasserstoffrest, an den ein durch ein oder zwei Halogenatome substituierter Phenylrest ankondensiert ist dergestalt, daß sich mit dem N-Atom ein fünf- oder sechsgliedriger Ring bildet, $R^3$ and $R^4$ unabhängig voneinander Wasserstoff, Methyl oder Äthyl und Z mindestens 1 Halogenatom bedeutet. Die Erfindung betrifft ferner die Verwendung der Verbindungen I zur Behandlung von Infektionen durch Helminthen, sowie neue Verbindungen der Formel I, bei denen $R^1$ einen durch eine $OCF_3$-Gruppe substituierten Phenylrest und $R^2 = H$ oder $R^2$ Alkyl bedeutet.

EP 0 216 028 A2

Croydon Printing Company Ltd.

HOECHST AKTIENGESELLSCHAFT Dr.AU/gm HOE 85/F 118

## Arzneimittel und deren Verwendung

Es wurde gefunden, daß bestimmte Anthranilsäurederivate sich zur Bekämpfung von Helminthen ausgezeichnet eignen. Diese Verbindungen sind größtenteils aus GB-PS 865,735 bekannt. Hierin werden jedoch lediglich insektizide Wirkungen beschrieben.

Gegenstand der vorliegenden Erfindung sind daher Arzneimittel, die mindestens eine Verbindung der Formel I

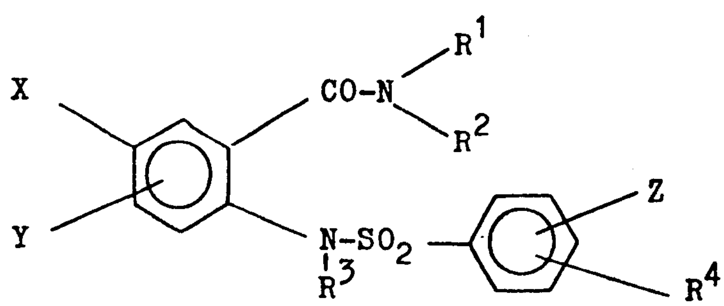

(I)

worin X Halogen, $CF_3$, $NO_2$, $SO_nR$ mit $R=CH_3$ oder $C_2H_5$, n=0-2 bedeutet, Y Wasserstoff oder Halogen, $R^1$ einen durch mindestens ein Halogenatom oder eine $CF_3$- oder $OCF_3$-Gruppe substituierten Phenylrest oder einen Alkylrest mit 6-18 Kohlenstoffatomen, $R^2$ Wasserstoff oder einen Alkylrest mit 1 bis 8 Kohlenstoffatomen bedeuten, oder $R^1$ und $R^2$ zusammen einen gesättigten oder äthylenisch ungesättigten Kohlenwasserstoffrest, an den ein durch ein oder zwei Halogenatome substituierter Phenylrest ankondensiert ist dergestalt, daß sich mit dem N-Atom ein fünf- oder sechsgliedriger Ring bildet, $R^3$ und $R^4$ unabhängig voneinander Wasserstoff, Methyl oder Äthyl und Z mindestens 1 Halogenatom bedeuten, enthalten.

Halogen bedeutet stets bevorzugt Fluor, Chlor oder Brom.

Bevorzugt sind solche Verbindungen, in denen X Chlor oder Brom, Y Wasserstoff oder Chlor, $R^1$ Phenyl, das durch ein bis drei Halogenatome aus der Gruppe Fluor, Chlor und Brom oder durch eine $CF_3$- oder $OCF_3$-Gruppe, vorzugsweise in 4-Stellung zur Aminofunktion, substituiert ist, $R^2$ Wasserstoff, Methyl oder Äthyl, $R^3$ und $R^4$ Wasserstoff und Z ein bis drei Halogenatome aus der Gruppe Fluor, Chlor und Brom bedeuten.

Insbesondere auch unter dem Gesichtspunkt der wirtschaftlichen Zugänglichkeit bevorzugt sind die Verbindungen, in denen X Chlor, Y Wasserstoff, $R^1$ ein durch ein oder zwei Chlor- oder Bromatome substituiertes Phenyl, $R^2$ bis $R^4$ Wasserstoff und Z ein oder 2 Chloratome bedeuten.

Für den Fall, daß $R^1$ und $R^2$ zusammen einen Kohlenwasserstoffrest bedeuten, entspricht dieser, wenn man das benachbarte Stickstoffatom mit einbezieht, insbesondere einem halogenierten Tetrahydrochinolin- oder Dihydroindol-Rest.

Die allgemeine Formel I enthält 3 Bauelemente: Eine substituierte Anthranilsäure, ein Amin $R^1R^2NH$ und eine durch Z substituierte Benzolsulfonsäure.

Beispiele für die substituierte Anthranilsäure sind: 2-Amino-5-chlor-benzoesäure, 2-Amino-3,5,-dichlor-benzoesäure, 2-Amino-4,5-dichlorbenzoesäure, 2-Amino-5,6-dichlorbenzoesäure, 5-Chlor-2-methylamino-benzoesäure, 5-Chlor-2-äthylamino-benzoesäure, 2-Amino-5-fluor-benzoesäure, 2-Amino-5-brom-benzoesäure, 2-Amino-3,5-dibrombenzoesäure, 2-Amino-5-brom-3-chlor-benzoesäure, 2-Amino-5-brom-4-chlor-benzoesäure, 2-Amino-3-brom-5-chlor-benzoesäure, 2-Amino-5-nitro-benzoesäure, 2-Amino-5-nitro-3-chlor-benzoesäure, 2-Amino-5-nitro-4-chlor-benzoesäure, 2-Amino-5-trifluormethyl-benzoesäure, 2-Amino-5-äthylthio-benzoesäure, 2-Amino-4-chlor-5-methylthio-benzoesäure, 2-Amino-5-äthylsulfonylbenzoesäure.

Als Beispiele für die Aminkomponente seien genannt: 4-Fluoranilin, 4-Chlor-anilin, 4-Brom-anilin, 4-Jod-anilin, 3,4-Dichloranilin, 2,4-Dichloranilin, 2,4,5-Trichloranilin, 2-Brom-4-chloranilin, 4-Brom-2-chloranilin, 4-Fluor-2-chloranilin, 4-Fluor-2-bromanilin, 3-Trifluormethyl-anilin, 4-Trifluormethyl-anilin, 4-Trifluormethoxy-anilin, 4-Chlor-N-äthyl-anilin, 2,4-Dichlor-N-äthylanilin, 3,4-Dichlor-N-methyl-anilin, 5-Chlor-indolin, 6-Chlor-1,2,3,4-tetrahydrochinolin, 1-Amino-octan, 1-Amino-dodecan, 1-Amino-octadecan, 1-(Äthylamino-)octan, 1-(Methylamino-)dodecan, Dihexylamin, Dioctylamin, Cyclooctylamin, Cyclododecylamin.

Beispiele für die Sulfonsäure-Komponente sind: 4-Fluor-benzolsulfonsäure, 4-Chlor-benzolsulfonsäure, 4-Brom-benzolsulfonsäure, 2-Chlor-benzolsulfonsäure, 3,4-Dichlor-benzolsulfonsäure, 2,4-Dichlor-benzolsulfonsäure, 2,5-Dichlorbenzolsulfonsäure, 3-Chlor-4-fluor-benzolsulfonsäure, 4-Chlor-3-methylbenzolsulfonsäure, 3-Chlor-4-methyl-benzolsulfonsäure, 2,4,5-Trichlorbenzolsulfonsäure.

Wichtige Vertreter der durch die Formel I definierten Verbindungsklasse sind z.B. 5-Chlor-2-[(4-chlor-phenylsulfonyl)-amino]-benzoesäure-(4-chloranilid), Schmp. 216°C (1); 5-Chlor-2-[(4-chlor-phenylsulfonyl)-amino]-benzoesäure-(2,4-dichlor-anilid), Schmp. 173°C (2); 5-Chlor-2-[(4-chlor-phenylsulfonyl)-amino]-benzoesäure-(3,4-dichloranilid), Schmp. 244°C (3); 5-Chlor-2-[(4-chlor-phenylsulfonyl)-amino]-benzoesäure-(4-fluoranilid),

Schmp. 266°C (4); 5-Chlor[(4-chlor-phenylsulfonyl)-amino]-benzoesäure-(4-brom-anilid), Schmp. 210°C (5); 5-Chlor-2-[4-chlorphenylsulfonyl)-amino]-benzoesäure-(4-chlor-N-methylanilid), Schmp. 183°C (6); 5-Chlor-2-[(4-chlor-phenylsulfonyl)-amino]-benzoesäure-(3-trifluormethyl-anilid), Schmp. 216°C (7); 5-Chlor-2-[(4-chlor-phenylsulfonyl)-amino]-benzoesäure-(4-jod-anilid), Schmp. 229°C (8); 5-Brom-2-[(4-chlor-phenylsulfonyl)-amino]-benzoesäure-(4-chlor-anilid), Schmp. 210°C (9); 5-Brom-2-[(4-chlor-phenylsulfonyl)-amino]-benzoesäure-(2,4-dichlor-anilid), Schmp. 173°C (10); 5-Brom-2-[(4-chlor-phenylsulfonyl)-amino]-benzoesäure-(3-trifluormethyl-anilid), Schmp. 220°C (11); 4,5-Dichlor-2-[(4-chlor-phenylsulfonyl)-amino]-benzoesäure-(4-chlor-anilid), Schmp. 207°C (12); 3,5-Dichlor-2-[(4-chlor-phenylsulfonyl)-amino]-benzoesäure-(4-chlor-anilid), Schmp. 260°C (13); 3,5-Dichlor-2-[(4-chlor-phenylsulfonyl)-amino]-benzoesäure-(4-fluor-anilid), Schmp. 256°C (14); 3,5-Dichlor-2-[(4-chlor-phenylsulfonyl)-amino]-benzoesäure-(4-chlor-N-äthyl-anilid), Schmp. 163°C (15); 3,5-Dichlor-2-[(4-chlor-phenyl-sulfonyl)-amino]-benzoesäure-dodecylamid, Schmp. 193°C (16); 5-Chlor-2-[(4-chlor-phenylsulfonyl)-amino]-benzoesäure-dodecylamid, Schmp. 117°C (17); 5-Chlor-2-[(4-chlor-phenylsulfonyl)-amino]-benzoesäure-octadecylamid, Schmp. 108°C (18); 5-Chlor-2-[(4-chlor-phenylsulfonyl)-amino]-benzoesäure-(N-methyl-dodecylamid), Schmp. 50°C (19); 5-Chlor-2-[(4-brom-phenylsulfonyl)-amino]-benzoesäure-(4-chlor-anilid), Schmp. 217°C (20); 5-Chlor-2-[(4-brom-phenylsulfonyl)-amino]-benzoesäure-(4-brom-anilid), Schmp. 209°C (21); 5-Chlor-2-[(4-fluor-phenylsulfonyl)-amino]-benzoesäure-(4-chloranilid), Schmp. 207°C (22); 2-Chlor-2-[(4-fluorphenylsulfonyl)-amino]-benzoesäure-(4-fluor-anilid), Schmp. 225°C (23); 5-Chlor-2-[(4-fluor-phenylsulfonyl)-amino]-benzoesäure-(3,5-dichloranilid), Schmp. 250°C (24); 5-Chlor-2-[(2,4-dichlor-phenylsulfonyl)-amino]-benzoesäure-(4-chloranilid), Schmp. 224°C (25); 5-Chlor-2-[(3,4-dichlor-phenylsulfonyl)-amino]-benzoesäure-(4-chlor-anilid), Schmp. 225°C (26); 5-Chlor-2-[(3,4-dichlor-phenyl-sulfonyl)-amino]-benzoesäure-octylamid, Schmp. 145°C (27); 5-Chlor-2-[(3,4-dichlor-phenyl-sulfonyl)-amino]-benzoesäure-(4-fluor-anilid), Schmp. 230°C (28); 5-Chlor-2-[(3,4-dichlor-phenylsulfonyl)-amino]-benzoesäure-(4-chlor-N-äthyl-anilid), Schmp. 136°C (29); 5-Chlor-2-[(3,4-dichlor-phenylsulfonyl)-methylamino]-benzoesäure-(4-chlor-anilid), Schmp. 151°C (30); 5-Chlor-2-[(3,4-dichlor-phenylsulfonyl)-amino]-benzoesäure-dodecylamid, Schmp. 130°C (31); 5-Chlor-2-[(3,4-dichlor-phenyl-sulfonyl)-amino]-benzoesäure-octa-decylamid,Schmp. 120°C (32); 5-Chlor-2-[(3,4-dichlor-phenyl-sulfonyl)-amino]-benzoesäure-(N-methyl-dodecylamid), Schmp. 52°C (33); 5-Chlor-2-[(3,4-dichlor-phenylsulfonyl)-amino]-benzoesäure-(4-trifluormethyl-anilid), Schmp. 225°C (34); 5-Chlor-2-[(3,4-dichlorphenylsulfonyl)-amino]-benzoesäure-(4-trifluormethoxy-anilid), Schmp. 200°C (35); 2-[(4-Chlor-phenylsulfonyl)-amino]-5-nitro-benzoesäure-(4-chlor-anilid), Schmp. 215°C (36); 2-[(4-Chlor-phenylsulfonyl)-amino]-5-nitrobenzoesäure-(3-trifluoromethyl-anilid), Schmp. 218°C (37); 2-[(3,4-Dichlor-phenylsulfonyl)-amino]-5-nitro-benzoesäure-(4-chlor-anilid), Schmp. 228°C (38); 2-[(3,4-Dichlor-phenyl-sulfonyl)-amino]-5-äthylthio-benzoesäure-(4-chloranilid), Schmp. 169°C (39); 2-[(3,4-Dichlor-phenyl-sulfonyl)-amino]-5-äthylsulfonyl-benzoesäure-(4-chlor-anilid), Schmp. 195°C (40); 5-Chlor-2-[(3,4-dichlor-phenyl-sulfonyl)-amino]-benzoesäure-cyclododecylamid, Schmp. 192°C, (41); 5-Chlor-2-[(3,4-dichlor-phenylsulfonylmethylamino]-benzoesäure-(4-chlor-N-äthyl-anilid), Schmp. 122°C (42).

2-(3,4-Dichlor-phenylsulfonyl-amino)-5-fluor-benzoesäure-(4-chlor-anilid), Schmp. 227°C (43)

2-(3,4-Dichlor-phenylsulfonyl-amino)-5-fluor-benzoesäure-(4-chlor-N-äthyl-anilid), Schmp. 120°C (44)

2-(3,4-Dichlor-phenylsulfonyl-amino)-5-fluor-benzoesäure-dodecylamid, Schmp. 134°C (45)

2-(3,4-Dichlor-phenylsulfonyl-amino)-5-fluor-benzoesäure-(4-fluor-anilid), Schmp. 216°C (46)

Ausserdem:

5-Chlor-2-[(3,4-dichlor-phenylsulfonyl)-äthylamino]-benzoesäure-(4-chlor-anilid), Schmp. 130°C (47)

3,5-Dichlor-2-(3,4-dichlor-phenylsulfonyl-amino)-benzoesäure-(4-trifluormethoxy-anilid), Schmp. 260°C (48)

3,5-Dichlor-2-(3,4-dichlor-phenylsulfonyl-amino)-benzoesäure-(4-chlor-N-äthyl-anilid), Schmp. 173°C (49)

3,5-Dichlor-2-(3,4-dichlor-phenylsulfonyl-amino)-benzoesäure-dodecylamid, Schmp. 197°C (50)

3,5-Dichlor-2-(3,4-dichlor-phenylsulfonyl-amino)-benzoesäure-(4-chlor-anilid), Schmp. 260°C (51)

Die nachgeordneten Zahlen in dieser Aufstellung beziehen sich auf die Tabellen 1 bis 3 zur Wirksamkeit dieser Verbindungen. Als isomere, nicht unter den Anspruch fallende Vergleichsverbindungen V1-10 wurden die nachfolgend aufgeführten synthetisiert und geprüft:

4-Chlor-2-[(4-chlor-phenylsulfonyl)-amino]-benzoesäure-(4-chloranilid), Schmp. 224°C (V1); 2-Chlor-4-[(4-chlor-phenylsulfonyl)-amino]-benzoesäure-(4-chlor-anilid), Schmp. 185°C (V2); 3-Chlor-4-[(4-chlor-phenylsulfonyl)-amino]-benzoesäure-(4-chlor-anilid), Schmp. 154°C (V3); 4-Chlor-3-[(4-Chlorphenylsulfonyl)-amino]-benzoesäure-(4-chlor-anilid), Schmp. 206°C (V4); 2-Chlor-5-[(4-chlor-phenylsulfonyl)-amino]-benzoesäure-(4-chlor-anilid), Schmp. 182°C (V5); 4-Chlor-2-[(4-chlor-phenylsulfonyl)-amino]-benzoesäure-(3,4-dichlor-anilid), Schmp. 228°C (V6); 2-Chlor-5-[(4-chlor-phenylsulfonyl)-amino]-benzoesäure-(4-fluor-anilid), Schmp. 194°C (V7); 2-Brom-4-[(4-chlor-phenylsulfonyl)-amino]-benzoesäure-(4-chlor-anilid), Schmp. 215°C (V8); 4-Brom-2-[(4-chlor-phenylsulfonyl)-amino]-benzoesäure-(4-chlor-anilid), Schmp. 214°C (V9); 2-Chlor-4-[(4-chlor-phenylsulfonyl)-amino]-benzoesäure-(3,4-dichlor-anilid), Schmp. 201°C (V10).

Die erfindungsgemäß zu verwendenden Verbindungen sind zum Teil bereits aus der britischen Patentschrift 865,735 bekannt. In dieser fehlt jedoch jeder Hinweis auf eine Wirksamkeit der Verbindungen gegen Helminthen, insbesondere Leberegel. Zudem ist offensichtlich die Wirksamkeit gegen Insekten wenig strukturspezifisch und sehr stark auf den Halogengehalt abgestellt, was daraus hervorgeht, daß der Halogengehalt nur als Summe der Atome bzw. Trifluormethylgruppen ohne Rücksicht auf die Position im Molekül angegeben wird. Demgegenüber sind für die anthelmintische Wirksamkeit wesentlich engere strukturelle Bedingungen gegeben, wie sie durch die allgemeine Formel I beschrieben werden. Isomere Derivate der 3- bzw. 4-Aminobenzoesäuren sind deutlich schwächer als die der Anthranilsäure, wie sich aus der Tabelle 3 ergibt. Weiterhin ist der Substituent X in 5-Position wesentlich und schließlich kommen als Aminkomponente der Carbonamidstruktur nicht nur substituierte Aniline, sondern auch hinreichend langkettige aliphatische Amine in Betracht.

Die Herstellung der erfindungsgemäß zu verwendenden Verbindungen kann nach üblichen Methoden erfolgen, z.B. durch Acylierung der entsprechend substituierten Anthranilsäuren mit den Sulfochloriden, Umwandlung der Carboxylgruppen in die Säurechloride und Umsetzung mit den Aminen, s. jeweils GB-PS 865,735; EP-A 148,725 oder durch Überführung entsprechend substituierter 2-Nitro-benzoesäuren in die entsprechenden Amide, Reduktion der Nitrogruppe und Acylierung mit den Sulfochloriden, s. EP-A 148,725, oder durch Austausch des Halogens in 5-Halogen-2-nitro-benzoesäure-amiden mit Nucleophilen, Reduktion und Acylierung, oder durch Kernchlorierung der im Sulfonsäurerest entsprechend substituierten 2-Sulfonylamino-benzoesäuren bzw. deren Estern und nachfolgende Umwandlung in die Amide, oder Derivatierung von Verbindungen der Formel I wie die Oxidation von Thioethern (X=SR).

Insbesondere sind die folgenden Verfahren von Bedeutung:

a) Umsetzung der Verbindungen der Formel II

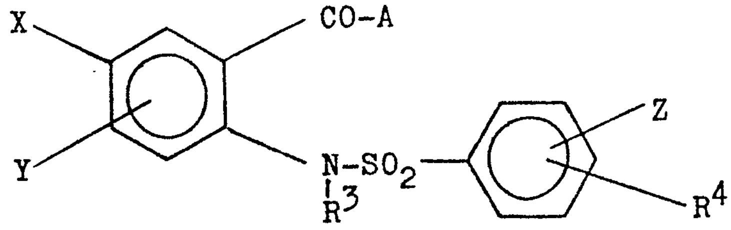

(II)

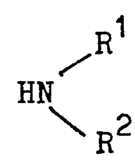

(III)

mit Verbindungen der Formel (III), wobei A in Formel (II) OH oder eine reaktive Abgangsgruppe wie Halogen, Tosyloxy oder einen aktivierten Esterrest wie Alkoxycarbonyloxy bedeutet. Diese Umsetzung wird in einem geeigneten inerten Lösungsmittel wie Benzol, Toluol, $CH_2Cl_2$, Dimethylformamid, Tetrahydrofuran, Aceton, Acetonitril, gegebenenfalls in Gegenwart einer Base wie Triethylamin, Pyridin, Picolin zwischen 0°C bis 150°C. Wenn A= OH bedeutet kann die Reaktion in Gegenwart eines Kondensationsmittels wie Dicyclohexylcarbodiimid durchgeführt werden.

b) Umsetzung der Verbindungen der Formel (IV)

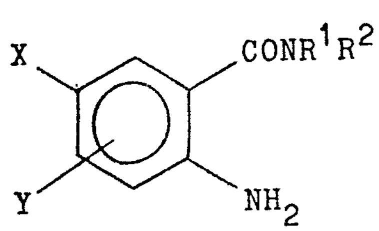

(IV)

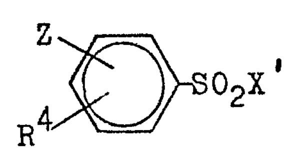

(X' = Halogen)

(V)

mit Verbindungen der Formel V und gegebenenfalls anschließende Alkylierung des Sulfonylstickstoffatoms, vgl EP-A 148,725. Die Umsetzung wird analog wie unter a) beschrieben durchgeführt, (Temperaturbereich 0°C bis 100°C), wobei ebenfalls die obengenannten Basen zugesetzt werden können; es kann auch Pyridin als Lösungsmittel verwendet werden.

Die Ausgangsverbindungen der Formeln (II) bis (V) können nach dem Fachmann bekannten Methoden, beispielsweise, wie sie in EP-A 148,725 beschrieben sind, hergestellt werden.

Die Verbindungen der allgemeinen Formel I gemäß der Erfindung sind wertvolle Chemotherapeutika. Sie wirken gegen Helminthen, insbesondere Trematoden, Zestoden und Nematoden. Besonders ausgeprägt ist ihre Wirksamkeit gegen Leberegel, z. B. gegen den großen Leberegel Fasciola hepatica, der als Parasit von Nutztieren wie Schafen und Rindern weit verbreitet ist. Weidetiere sind häufig von Bandwürmern und in besonderem Maße von Nematoden befallen. Die Verseuchung einer Herde mit diesen Helminthen führt zu empfindlichen wirtschaftlichen Schäden. Deshalb kommt einem spezifischen Therapeutikum gegen die genannten drei Helminthen-Gruppen eine erhebliche Bedeutung zu.

Der anthelminthische Effekt der Verbindungen der Formel I läßt sich sowohl an Laboratoriumstieren als auch an Haustieren zeigen. Die Wirkstoffe werden auf einmal oder in einer Mehrzahl kleiner Dosen ggf. über einen längeren Zeitraum verteilt, zusammen mit einem geeigneten pharmazeutischen Lösungsmittel bzw. Trägerstoff oral oder parenteral, z.B. auch mit der "pour-on" Methode, vgl. z.B. EP-A 45424 appliziert. Die Applikationsart ist je nach Lage des Falles zu wählen. Die Anwendungskonzentration liegt

bei parenteraler Gabe im Bereich von 0,1 - 5,0 mg/kg Körpergewicht bzw. bei oraler Gabe im Bereich von 0,5 - 50 mg/kg Körpergewicht.

Zur oralen Applikation sind feste oder flüssige Verabreichungsformen wie Tabletten, Dragees, Boli, Pulver, Granulate oder Mischungen der Wirkstoffe mit Futter möglich; auch Arzneitränke können verwendet werden. Zur parenteralen Applikation verwendet man flüssige oder feste Zubereitungen wie Emulsionen, Suspensionen, Lösungen oder Implantate der Wirkstoffe. Zur Herstellung dieser Zubereitungen kommen übliche, physiologisch unbedenkliche Hilfs- und Trägerstoffe in Frage wie Talk, Milchzucker, Magnesiumstearat, anorganische Polymere wie hochdisperses Siliziumdioxid, Alumosilikate, Polyphosphate, organische Polymere, wie Polyglykolsäure, Polymilch- oder Polybuttersäure oder deren Copolymere, Zellulose und deren Derivate, Polyacrylsäuren, Polyvinylpyrrolidon, Poly(vinyl oder ähnliche) alkohole, Polyäthylenglykol, ferner Paraffinöl, Lösemittel, Wasser, ferner übliche Konservierungsmittel oder Puffersubstanzen.

Flüssige Zubereitungen enthalten im allgemeinen 1-50 Gew.-% Wirkstoff, feste Zubereitungen im allgemeinen 1-80 Gew.-% Wirkstoff.

Eine flüssige Zubereitung wie eine Suspension wird beispielsweise hergestellt, in dem man zu einer Trägerlösung (enthaltend beispielsweise das lösliche organische Polymer, Konservierungsstoffe, Puffersubstanzen, Lösungsmittel) eine Mischung aus Siliciumdioxid (oder eines Alumosilikats) und dem Wirkstoff hinzugibt und dieses Gemisch auf geeignete Weise (z.B. Schütteln) homogenisiert. Eine solche Zubereitung enthält beispielsweise 6 % Wirkstoff, 3 % $SiO_2$, 3 % eines Cellulosederivats, 3 % eines Puffers (z.B. eines Zitrats), 1 % Konservierungsmittel und 84 % Wasser. (% $\hat{=}$ Gew.-%).

Zur Feststellung der anthelminthischen Wirkung der Verbindungen der Formel I wurden chemotherapeutische Untersuchungen an ca. 25-30 kg schweren Schaflämmern ausgeführt, die entweder mit Metazerkarien von Fasciola hepatica oder mit Larven von Magen-Darm-Strongyliden, vornehmlich Haemonchus contortus und Trichostongylus colubriformis, oral infiziert worden waren. Nach Ablauf der Präpatenzperiode (Zeit zwischen Infektion und Geschlechtsreife der Parasiten mit beginnender Ausscheidung von Eiern oder Larven) wurde der Parasitenbefall durch Kotuntersuchungen gesichert. Im Fall der Leberegel-Infektion erfolgte der qualitativ-quantitative Nachweis von Eiern nach der Telemann-Anreicherung (Deutsche Medizinische Wochenschrift 34 : 1510, (1908)). Die Eizahl pro Gramm Kot von Magen-Darm-Strongyliden wurde im modifizierten McMaster-Verfahren nach Wetzel (Tierärztliche Umschau 6, 209, (1951)) bestimmt. Unmittelbar danach wurde die Behandlung der Schafe (im allgemeinen 3 - 4 Tiere pro Wirkstoff, mindestens aber 2) vorgenommen. Den Tieren wurde oral oder parenteral eine Suspension von unterschiedlicher Wirkstoffmenge in jeweils 10 ml einer 1%igen Carboxymethylcellulose-Suspension appliziert. Jeweils am 7., 14. und 28. Tag nach der Behandlung wurde wiederum nach den vorstehend angegebenen Verfahren die Eizahl pro Gramm Kot ermittelt und ihre prozentuale Abnahme im Vergleich zum Ausgangswert vor der Behandlung errechnet. Mit einigen Verbindungen wurden außerdem beim Fasciola-Befall noch eine zusätzliche Prüftechnik angewandt: Nach Abschluß der Präpatenzperiode wurde bei Fasciola-positiven Schafen ein künstlicher Gallengang geschaffen, der am Ausgang der Gallenblase ein Sieb enthielt, so daß die nach der Behandlung abgetöteten Leberegel in der Gallenblase aufgefangen werden konnten. Vier bis sieben Tage nach der Behandlung wurden die so operierten und dann behandelten Schafe getötet, die abgetöteten Leberegel in der Gallenblase sowie noch eventuell in der Leber vorhandene lebende Leberegel gezählt und von der gesamten Wurmbürde der Prozentsatz der abgetöteten Parasiten berechnet.

Zusätzlich sind auch Wirksamkeitstest bei Helminthen während der Präpatenzperiode möglich. Hierbei werden die Wirkstoffe zu bestimmten Zeitpunkten innerhalb dieser Periode appliziert, bei denen der Übergang vom einen zum nächsten Entwicklungsstadium erfolgt.

Von den Verbindungen der Formel I sind neu diejenigen Stoffe, bei denen $R^2$ einen Alkylrest mit 1 bis 8 C-Atomen oder solche, bei denen $R^1$ einen durch eine $OCF_3$-Gruppe substituierten Phenylrest und $R^2$ = H bedeutet. Diese Verbindungen sind ebenfalls Gegenstand der vorliegenden Erfindung.

In den nachfolgenden Tabellen 1 und 2 sind die Wirkungen von Verbindungen der Formel I und von bekannten handelsüblichen Vergleichsprodukten aufgeführt. Als Vergleichsprodukte dienten 3'-Chlor-4'-(p-chlor-phenoxy)-3,5-dijod-salicylanilid (Rafoxanide ) und 5,5'-Dichlor-2-2'-dihydroxy-3,3'-dinitro-biphenyl (Niclofolan).

In der Tabelle 3 werden die Wirksamkeiten von erfindungsgemäßen 5-Halogen-benzoesäureaniliden mit denen entsprechender Derivate isomerer nicht beanspruchter Halogen-aminobenzoesäuren verglichen.

Aus den Tabellen ist eine Überlegenheit der erfindungsgemäßen Sulfonylanthranilsäureanilide, meistens in mehreren, mindestens aber in einem der folgenden Punkte ersichtlich:

1. geringere Dosierung (Dosis curativa minima/D.c.m.)
2. bessere Wirksamkeit (Effekt)
3. breiteres Spektrum
4. größerer chemotherapeutischer Index

Der chemotherapeutische Index gibt den Quotienten aus Dosis tolerata maxima und Dosis curativa minima an.

Die typischen Verfahrensweisen für die Herstellung der erfindungsgemäß verwendeten Produkte der allgemeinen Formel I werden durch die folgenden Beispiele erläutert.

Beispiele:

1 : 5-Brom-2-[(4-chlor-phenylsulfonyl)-amino]-benzoesäure-(3-trifluormethyl-anilid)

Zu einer Lösung von 69 g 2-Amino-5-brom-benzoesäure-methylester in 150 ml Pyridin gab man unter Eiskühlung und gutem Rühren portionsweise 63 g gepulvertes 4-Chlor-benzolsulfochlorid, rührte anschließend noch 12 Stunden bei Raumtemperatur nach, goß auf Wasser und Eis, dekantierte ab, behandelte das organische Produkt mit verdünnter Salzsäure, zerkleinerte, saugte ab, wusch in Wasser nach und trocknete. Ausbeute 115 g an praktisch sauberen 5-Brom-2-[(4-chlor-phenylsulfonyl)-amino]-benzoesäure-methylester vom Schmp. 104°C. 45 g dieses Esters erhitzte man in der Lösung 9,8 g NaOH in einer Mischung aus 20 ml Wasser und 300 ml Methanol 4 Stunden unter Rückfluß, löste das ausgefallene Natriumsalz durch Zugabe von 1 l Wasser auf, stellte mit konzentrierter Salzsäure sauer, saugte die Ausfällung ab, wusch mit Wasser und trocknete. Ausbeute 39,9 g, Schmp. 225°C. Die rohe Säure erhitzte man in 100 ml Thionylchlorid 1 Stunde unter Rückfluß und entfernte anschließend den Überschuß im Vakuum der Wasserstrahlpumpe. Das rohe Säurechlorid wog 42,6 g und schmolz bei ca. 125 °C. Zu einer Lösung von 20 g dieses Säurechlorids und 8,9 g 3-Trifluormethyl-anilin in 200 ml Methylenchlorid tropfte man unter Eiskühlung 6,1 g Triäthylamin, rührte noch 24 Stunden bei Raumtemperatur nach, saugte die Ausfällung ab, wusch mit verdünnter Salzsäure und Wasser und trocknete. Die Ausbeute des bei 220°C schmelzenden Anilids betrug 19,1 g. Das Produkt

war fast sauber, konnte jedoch auch noch mit geringem Verlust aus Acetonitril umkristallisiert werden.

2 : 5-Chlor-2-[(4-chlor-phenylsulfonyl)-amino]-benzoesäure-dodecylamid

Zu einer Lösung von 28,2 g 5-Chlor-2-nitro-benzoylchlorid in 120 ml Methylenchlorid tropfte man unter Eiskühlung eine Lösung von 27,8 g Dodecylamin und 17,7 g Triäthylamin in 120 ml Methylenchlorid, rührte noch 24 Stunden bei Raumtemperatur, wusch mit verdünnter Salzsäure und mit Wasser und destillierte das Lösungsmittel unter vermindertem Druck ab. Man erhielt 43,6 g praktisch sauberes Produkt vom Schmp. 110°C. 37,4 g dieser Verbindung wurden in 400 ml Methanol gelöst und mit Raney-Nickel bei Raumtemperatur unter Normaldruck hydriert. Innerhalb von 3 Stunden wurde die berechnete Menge Wasserstoff aufgenommen. Man filtrierte heiß vom Katalysator ab, dampfte das Filtrat unter vermindertem Druck ein und kristallisierte den Rückstand aus Methanol um. Ausbeute 28 g, Schmp. 113°C.

Man löste 17 g dieser Aminoverbindung in 50 ml Pyridin, gab unter Rühren und Eiskühlung portionsweise 11 g gepulvertes 4-Chlor-benzolsulfochlorid zu, rührte noch 24 Stunden bei Raumtemperatur nach, gab das Gemisch dann auf 1 l Eiswasser, dekantierte die wäßrige Phase ab, behandelte das organische Material mit verdünnter Salzsäure, saugte ab, wusch mit Wasser, trocknete und kristallisierte aus Methanol um. Die Ausbeute an reinem Produkt betrug 20,2 g, der Schmp. 117°C.

3 : 2-[(3,4-Dichlor-phenylsulfonyl)-amino]-5-äthylthio-benzoesäure-(4-chlor-anilid)

Zu einer Lösung von 108 g 5-Chlor-2-nitrobenzoesäuremethylester und 35 g Äthylmercaptan in 170 ml Dimethylsulfoxid

gab man unter guter Kühlung bei 0°C portionsweise 57 g K-tert.-Butanolat. Dabei erfolgte jeweils eine heftige Reaktion. Nach 24 Stunden versetzte man mit Wasser, saugte die Ausfällung ab, wusch mit Wasser, trocknete, verrührte mit 400 ml Methanol, kühlte auf -78°C, saugte ab, wusch mit tiefgekühltem Methanol und trocknete. Die Ausbeute an dem sauberen Thioäther vom Schmp. 71°C betrug 91,8 g. 40 g dieser Verbindung löste man in 350 ml Methanol und fügte eine Lösung von 16,6 g NaOH in 40 ml Wasser hinzu. Nach 24 Stunden bei Raumtemperatur destillierte man das Lösungsmittel unter vermindertem Druck ab, nahm den Rückstand in Wasser auf, säuerte mit Salzsäure an, saugte ab, wusch mit Wasser und trocknete. Ausbeute 34,1 g, Schmp. 142°C.

Man erhitzte die rohe Säure mit 100 ml Thionylchlorid 3 1/2 Stunden unter Rückfluß, destillierte den Überschuß unter vermindertem Druck ab, versetzte den Rückstand zweimal mit je 100 ml Toluol und destillierte jeweils unter vermindertem Druck ab. Der Rückstand betrug 37,8 g. Die gesamte Menge des Säurechlorids löste man in 100 ml Methylenchlorid und tropfte unter Eiskühlung eine Lösung von 22,3 g 4-Chlor-anilin und 20,2 g Triäthylamin in 75 ml Methylenchlorid zu. Nach 24 Stunden Rühren bei Raumtemperatur wusch man mit verdünnter Salzsäure und mit Wasser, trocknete, destillierte das Lösungsmittel ab und kristallisierte den Rückstand aus 300 ml Toluol um. Ausbeute 38,3 g, Schmp. 161°C.
Man löste 30 g des Anilids in einer Mischung von 250 ml Methanol und 50 ml Dimethylformamid und hydrierte mit Raney-Nickel bei etwa 40°C unter Normaldruck. Innerhalb von 2 Stunden wurde die berechnete Menge Wasserstoff aufgenommen. Man filtrierte heiß vom Katalysator ab, entfernte das Lösungsmittel unter vermindertem Druck, behandelte den Rückstand mit Wasser, saugte ab, wusch mit Wasser und trocknete. Das Produkt war fast sauber, die Ausbeute betrug 25,2 g, Schmp. ca. 130°C. Man löste 24,5 g dieses Amins in 70 ml Pyridin und tropfte unter Eiskühlung 20,9 g 3,4-Dichlorbenzolsulfochlorid zu. Nach 2 Tagen Rühren bei Raumtemperatur gab man auf Eis und Wasser, dekantierte, behandelte mit verdünnter Salzsäure, saugte ab, wusch mit Wasser, trocknete und kristallisierte aus Acetonitril um. Man erhielt 30,2 g reines Produkt vom Schmp. 169°C.

4 : 2-[(3,4-Dichlorphenylsulfonyl)-amino]-5-äthylsulfonylbenzoesäure-(4-chlor-anilid)

Man löste 8 g der nach Beispiel 3 gewonnenen Verbindung in 110 ml Eisessig, erhitzte auf 60°C und gab 5,8 g und nach 17 Stunden weitere 2,5 g 35%iges Wasserstoffperoxid zu. Nach insgesamt 21 Stunden Reaktionszeit destillierte man das Lösungsmittel unter vermindertem Druck ab und kristallisierte den Rückstand aus Toluol um. Ausbeute 7,0 g, Schmp. 195°C.

5 : 5-Chlor-2-[(3,4-dichlor-phenylsulfonyl)-amino]-benzoesäure-(4-chloranilid)

Zur Lösung von 390 g Natriumcarbonat in 2,25 l Wasser gab man bei 60°C portionsweise 205,5 g Anthranilsäure. Nach Auflösung tropfte man innerhalb von 30 Minuten bei der gleichen Temperatur 442 g 3,4-Dichlor-benzolsulfochlorid zu, rührte 2 Stunden nach, filtrierte heiß und versetzte das Filtrat unter Rühren vorsichtig mit Salzsäure, bis ein pH-Wert von 3 erreicht wurde. Die Ausfällung war erst harzig, wurde aber nach einiger Zeit kristallin. Man verrieb mit Wasser, saugte ab, wusch mit Wasser und trocknete. Die Ausbeute betrug 426 g, der Schmp. 174°C. Das Produkt war fast rein und konnte direkt weiterverarbeitet werden. In eine Lösung von 50 g dieser Säure in 130 ml Eisessig leitete man bei Rückflußtemperatur innerhalb von

drei Stunden 22 g Chlor ein, kühlte dann auf Raumtemperatur, saugte die Kristalle ab, wusch mit Eisessig und kaltem Methanol und trocknete. Die Ausbeute an der bei 226°C schmelzenden Chlorverbindung betrug 32,1 g; sie kann durch Aufarbeiten der Mutterlauge erhöht werden.
Man erhitzte 49 g dieser Säure mit 150 ml Thionylchlorid 3 Stunden zum Rückfluß, destillierte den Überschuß ab, schüttelte den Rückstand zweimal mit je 100 ml Oktan und destillierte jeweils unter vermindertem Druck ab. Man erhielt das Säurechlorid in einer Ausbeute von 51,3 g. Zu einer Lösung von 17,6 g dieser Verbindung und 6,8 g 4-Chlor-anilin in 150 ml Methylenchlorid tropfte man unter Eiskühlung eine Mischung aus 6,1 g Triäthylamin und 30 ml Methylenchlorid. Nach 24 Stunden Nachrühren bei Raumtemperatur wusch man mit verdünnter Salzsäure und Wasser, trocknete, destillierte das Lösungsmittel ab und kristallisierte den Rückstand aus Acetonitril um.
Die Ausbeute an der reinen Verbindung vom Schmp. 225°C betrug 11,6 g.

<u>Beispiele für Verbindungen 29, 35</u>

Ersetzte man unter sonst gleichen Reaktionsbedingungen in der letzten Verfahrensstufe von Beispiel 5 4-Chloranilin durch 4-Trifluormethoxy-anilin, so erhielt man die Verbindung 35, mit 4-Chlor-N-äthyl-anilin die Verbindung 29.

Tabelle 1: Chemotherapeutische Untersuchungen
(>90% Reduktion der Wurmbürde)
Wirtstier: Schaf
Parasit: Leberegel (Fasciola hepatica)
Dosis: mg/kg Körpergewicht, p.o.

| Verbindung Nr. | Dosis | Chemotherapeut. Index |
|---|---|---|
| 1 | 1,5 | über 25 |
| 2 | 7,5 | 8 |
| 3 | 5 | 10 |
| 4 | 2,5 | 20 |
| 5 | 5 | 10 |
| 9 | 5 | 10 |
| 12 | 5 | 10 |
| 13 | 5 | 10 |
| 14 | 7,5 | 5 |
| 17 | 2,5 | 20 |
| 19 | 7,5 | 5 |
| 20 | 2,5 | 20 |
| 25 | 7,5 | 6 |
| 26 | 1 | über 25 |
| 27 | 7,5 | 8 |
| 29 | 1,5 | über 25 |
| 30 | 5 | 10 |
| 32 | 5 | 10 |
| 34 | 2,5 | 20 |
| 35 | 2,5 | 20 |
| 40 | 5 | 10 |
| 42 | 2,5 | 20 |
| Rafoxanide | 7,5 | 6 |
| Niclofolan | 3 | 3 |

Tabelle 2: Chemotherapeutische Untersuchungen (>90% Reduktion der Wurmbürde)
Wirtstier: Schaf;
Parasit: Magen-Darm-Würmer(Ostertagia, Haemonchur, Trichostrongylus, Cooperia, jeweils verschiedene Spezies)
Dosis: mg/kg Körpergewicht, oral bzw. subkutan

| Verbindung Nr. | Dosis | Chemotherapeut. Index |
|---|---|---|
| 6 | 10 p.o. | 5 |
| 13 | 10 p.o. | 5 |
| 16 | 5 p.o. | 10 |
| 20 | 10 p.o. | 5 |
| 21 | 5 s.c. | über 6 |
| 22 | 10 p.o. | 5 |
| 33 | 5 s.c. | über 6 |
| 35 | 10 p.o. | 5 |
| 38 | 7,5 p.o. | 6 |
| 42 | 5 p.o. | 10 |
| Rafoxanide | 7,5 p.o.* | 6 |
| Niclofolan | keine Wirkung | - |

* Nur wirksam gegen Haemonchus contortus, aber unwirksam gegen die anderen Parasiten.

0216028

Tabelle 3: Vergleichende Untersuchung isomerer Sulfonyl-amino-benzoesäureanilide (>90% Reduktion der Wurmbürde)
Wirtstier: Schaf
Parasit: Leberegel (Fasciola hepatica)
Dosis: mg/kg Körpergewicht, p.o.

| Verbindung Nr. | Dosis | Chemotherapeut. Index |
|---|---|---|
| 1 | 1,5 | über 25 |
| V1 | 30 | 2 |
| V2 | 20 | 3 |
| V3 | 30 | 2 |
| V4 | 15 | 4 |
| V5 | 30 | 2 |
| 3 | 5 | 10 |
| V6 | 30 | 2 |
| 4 | 2,5 | 20 |
| V7 | 30 | 2 |
| 9 | 2,5 | 20 |
| V8 | 30 | 2 |
| V9 | 20 | 3 |
| 26 | 1 | über 25 |
| V10 | 10 | 5 |

PATENTANSPRÜCHE

1. Arzneimittel, gekennzeichnet durch einen Gehalt an mindestens einer Verbindung der Formel I

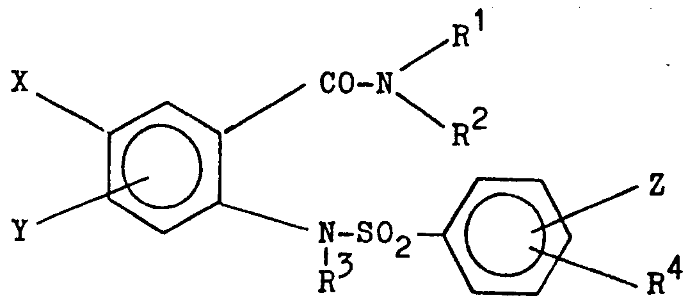

(I)

worin

X= Halogen, $CF_3$, $NO_2$, $SO_nR$ mit $R=CH_3$ oder $C_2H_5$, n=0-2
Y= Wasserstoff oder Halogen,
$R^1$= einen durch mindestens ein Halogenatom oder eine $CF_3$- oder $OCF_3$-Gruppe substituierten Phenylrest oder einen Alkylrest mit 6-18 Kohlenstoffatomen,
$R^2$= Wasserstoff oder einen Alkylrest mit 1 bis 8 Kohlenstoffatomen oder
$R^1$ und $R^2$ zusammen einen gesättigten oder äthylenisch ungesättigten Kohlenwasserstoffrest, an den ein durch ein oder zwei Halogenatome substituierter Phenylrest ankondensiert ist dergestalt, daß sich mit dem N-Atom ein fünf- oder sechsgliedriger Ring bildet,
$R^3$, $R^4$= unabhängig voneinander Wasserstoff, Methyl oder Ethyl und Z mindestens 1 Halogenatom bedeutet.

2. Arzneimittel nach Anspruch 1, dadurch gekennzeichnet, daß Halogen Fluor, Chlor oder Brom bedeutet.

3. Arzneimittel nach Anspruch 1 oder 2, gekennzeichnet durch wenigstens eines der Merkmale, daß X Chlor oder Brom ist, Y Wasserstoff oder Chlor ist, $R^1$ Phenyl, das

durch 1 bis 3 Halogenatome aus der Gruppe Fluor, Chlor und Brom oder durch eine $CF_3$- oder $OCF_3$-Gruppe, vorzugsweise in 4-Stellung zur Aminofunktion, substituiert ist,

$R^2$ Wasserstoff, Methyl oder Äthyl, $R^3$ und $R^4$ Wasserstoff und Z ein bis drei Halogenatome bedeuten.

4. Arzneimittel nach einem oder mehreren der Ansprüche 1 bis 3, dadurch gekennzeichnet, daß X Chlor, Y Wasserstoff, $R^1$ ein durch ein oder zwei Chlor- oder Bromatome substituiertes Phenyl, $R^2$ bis $R^4$ Wasserstoff und Z ein oder zwei Chloratome bedeuten.

5. Arzneimittel nach einem oder mehreren der Ansprüche 1 bis 4, dadurch gekennzeichnet, daß X Chlor oder Brom ist.

6. Arzneimittel nach Anspruch 5, gekennzeichnet durch einen Gehalt an einer 5-Chlor-Verbindung der Gruppe 5-Chlor-2-[(4-chlor-phenylsulfonyl)-amino]-benzoesäure-(4-chlor-anilid), 5-Chlor-2-[(4-chlor-phenylsulfonyl)-amino]-benzoesäure-(3,4-dichloranilid), 5-Chlor-2-[(4-chlor-phenylsulfonyl)-amino]-benzoesäure-(2,4-dichloranilid), 5-Chlor-2-[(4-chlor-phenylsulfonyl)-amino]-benzoesäure-(4-brom-anilid), 5-Chlor-2-[(4-chlor-phenylsulfonyl)-amino]-benzoesäure-(4-fluor-anilid), 5-Chlor-2-[(3,4-dichlor-phenylsulfonyl)-amino]-benzoesäure (4-trifluormethyl-anilid), 5-Chlor-2-[(3,4-dichlorphenylsulfonyl)-amino]-benzoesäure-(4-trifluormethoxyanilid), 5-Chlor-2-[(3,4-dichlor-phenylsulfonyl)-amino]-benzoesäure-(4-chlor-N-äthyl-anilid), 5-Chlor-2-[(3,4-dichlor-phenylsulfonyl)-amino]-benzoesäure-(4-chlor-anilid).

7. Arzneimittel nach Anspruch 5, gekennzeichnet durch eine 5-Brom-Verbindung, nämlich 5-Brom-2-[(4-chlor-phenylsulfonyl)-amino]-benzoesäure-(4-chlor-anilid) bzw. 5-Brom-2-[(3,4-dichlor-phenyl-sulfonyl)-amino]-benzoesäure-(4-chlor-anilid).

8. Verwendung von Verbindungen der Formel I zur Behandlung von Infektionen durch Helminthen, insbesondere bei Haustieren, vor allem Wiederkäuern wie Schaf und Rind.

9. Verfahren zur Herstellung eines Arzneimittels gemäß einem oder mehreren der Ansprüche 1 bis 7, dadurch gekennzeichnet, daß man

a) eine Verbindung der Formel II

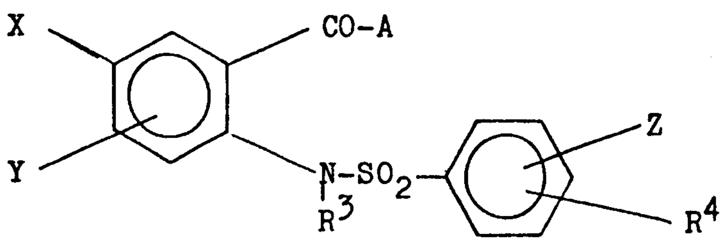

(II)

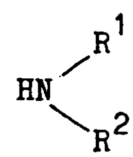

(III)

mit einer Verbindung der Formel (III), wobei A in Formel (II) OH oder eine reaktive Abgangsgruppe wie Halogen, Tosyloxy oder einen aktivierten Esterrest wie alkoxycarbonyloxy bedeutet, gegebenenfalls in Gegenwart einer Base zwischen 0°C und 150°C oder

b) eine Verbindung der Formel (IV)

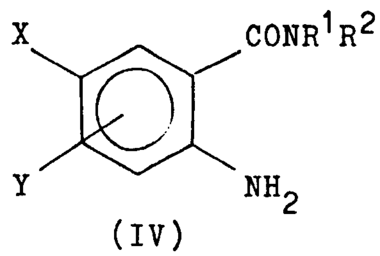

(IV)

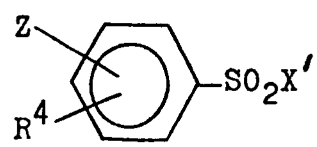

(X' = Halogen)

(V)

mit einer Verbindung der Formel V gegebenenfalls in Gegenwart einer Base bei Temperaturen zwischen 0°C und 100°C in an sich bekannter Weise umsetzt, und

c) anschließend die erhaltene Verbindung der Formel I in eine geeignete Darreichungsform überführt.

10. Verbindungen der Formel I, dadurch gekennzeichnet, daß $R^1$ einen durch eine $OCF_3$-Gruppe substituierten Phenylrest und $R^2$= H oder $R^2$ $(C_1-C_8)$Alkyl bedeutet und $R^1$ die in Anspruch 1 genannte Bedeutung besitzt.